(19) **Europäisches Patentamt / European Patent Office / Office européen des brevets**

(11) **EP 4 722 274 A1**

(12) **EUROPEAN PATENT APPLICATION**
published in accordance with Art. 153(4) EPC

(43) Date of publication:
08.04.2026 Bulletin 2026/15

(21) Application number: 24815580.6

(22) Date of filing: 30.05.2024

(51) International Patent Classification (IPC):
$C08J\ 3/12^{(2006.01)}$    $C08B\ 11/145^{(2006.01)}$
$C08J\ 9/42^{(2006.01)}$    $C12M\ 1/00^{(2006.01)}$

(52) Cooperative Patent Classification (CPC):
C08B 11/145; C08J 3/12; C08J 9/42; C12M 1/00

(86) International application number:
PCT/JP2024/019915

(87) International publication number:
WO 2024/248100 (05.12.2024 Gazette 2024/49)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 30.05.2023 JP 2023088825

(71) Applicant: ASAHI KASEI KABUSHIKI KAISHA
Tokyo 100-0006 (JP)

(72) Inventors:
• HARA, Yuichi
Tokyo 100-0006 (JP)
• HARANO, Kodai
Tokyo 100-0006 (JP)
• SANO, Ayae
Tokyo 100-0006 (JP)
• TERAKAWA, Misaki
Tokyo 100-0006 (JP)

(74) Representative: dompatent
Partnerschaft von
Patentanwälten und Rechtsanwälten mbB
Deichmannhaus am Dom
Bahnhofsvorplatz 1
50667 Köln (DE)

(54) **POROUS CELLULOSE PARTICLES AND METHOD FOR PRODUCING SAME**

(57) The present disclosure provides porous cellulose particles which are composed of cellulose, wherein: the surfaces of the porous cellulose particles have collagen; and the fixed amount of the collagen in a dry state is 10 μg/g to 25 mg/g inclusive (0.001-2.5 wt%) per 1 g of the porous cellulose particles.

EP 4 722 274 A1

**Description**

FIELD

The present invention relates to porous cellulose particles and to a method for producing them.

BACKGROUND

Efficient and large-scale culturing of cells, tissues and microorganisms is necessary in fields such as production of vaccines and antibody drugs, and regenerative medicine.

Methods using microcarriers are currently in wide use as mass cell culturing techniques for culturing of anchorage-dependent cells because they increase culture surface area with small volumes, while simultaneously making it possible to maintain high cell density per culture volume, as compared to monolayer culture methods which use flasks or roller bottles with lower culture surface area/volume ratios.

Cytodex 1 (product of Cytiva) particles having about 1.5 mmol/g charge capacity (dextran particles modified with cationic diethylaminoethyl groups) are widely used as microcarriers, but culturing of cells takes place only on the surfaces since the particles do not have a porous structure, and this has limited their proliferation potency. When agitation culture is carried out in a reactor, generation of shear stress has been a problem because it causes shedding of cells and significantly lowers culturing efficiency.

Porous microcarriers with large surface areas have been developed with an aim to solve these problems. PTL 1 describes culturing using porous cellulose particles exhibiting the feature of having mutually communicating voids with pore sizes larger than about 2 $\mu$m. Culturing using such porous particles allows cells to be cultured efficiently due to their large surface area, while also helping to avoid the effects of shear stress during agitation culture, since the cells are situated in the particle interiors.

[CITATION LIST]

[PATENT LITERATURE]

[PTL 1] Japanese Unexamined Patent Publication HEI No. 2-208331 (1990)

SUMMARY

[TECHNICAL PROBLEM]

With culturing microcarriers using the porous cellulose particles described in PTL 1, introduction of a charge onto the surface of the carrier causes cells to be adsorbed onto the surfaces of the porous microcarriers, but there is still room for improvement from the standpoint of cell adhesion and culturing efficiency.

In light of the current level of technology as described above, the problem to be solved by the invention is to provide porous cellulose particles with excellent cell adhesion, and allowing proliferation of cells with high efficiency.

[SOLUTION TO PROBLEM]

As a result of much diligent research on the problems described above, the present inventors have completed this invention after unexpectedly finding that the presence of collagen on the porous particle surfaces of porous cellulose reduces shedding of cells during high density culturing, and provides excellent cell proliferative ability (especially cell proliferative ability at the early stage of culturing).

Specifically, the present invention is as follows.

[1] Porous cellulose particles composed of cellulose,

having collagen on the porous cellulose particle surfaces,
wherein the fixed amount of the collagen in the dry state is 10 $\mu$g/g to 25 mg/g (0.001 to 2.5 wt%) per 1 g of porous cellulose particles.

[2] The porous cellulose particles according to [1] above, wherein the porous cellulose particles have numerous voids, with a mean opening size of 5 $\mu$m to 100 $\mu$m.

[3] The porous cellulose particles according to [1] or [2] above, wherein the porous cellulose particles form a continuous pore structure with adjacent voids mutually communicating by openings in the membranes dividing them.

[4] The porous cellulose particles according to any one of [1] to [3] above, wherein the mean particle size of the porous cellulose particles is 100 $\mu$m to 1000 $\mu$m.

[5] The porous cellulose particles according to any one of [1] to [4] above, wherein the sphericity of the porous cellulose particles is 0.7 or higher.

[6] The porous cellulose particles according to any one of [1] to [5] above, wherein the specific surface area of dry porous cellulose particles during drying is 0.3 $m^2$/g to 4.4 $m^2$/g.

[7] The porous cellulose particles according to any one of [1] to [6] above, wherein the fixed amount of the collagen in the dry state is 1 mg/g to 10 mg/g (0.1 to 1.0 wt%) per 1 g of porous cellulose particles.

[8] The porous cellulose particles according to any one of [1] to [7] above, wherein the porous cellulose particle surfaces are modified with a cationic substituent.

[9] The porous cellulose particles according to [8] above, wherein the cationic substituent is at least one selected from the group consisting of primary amino, secondary amino and tertiary amino groups.

[10] The porous cellulose particles according to any one of [1] to [9] above, wherein the charge capacity of the porous cellulose particles is 0 mmol/g to 5.0 mmol/g.

[11] The porous cellulose particles according to any one of [1] to [10] above, wherein the collagen is fiberized collagen.

[12] The porous cellulose particles according to any one of [1] to [10] above, wherein the collagen is crosslinked collagen.

[13] The porous cellulose particles according to any one of [1] to [12] above, wherein the cellulose composing the porous cellulose particles is copper-ammonia regenerated cellulose.

[14] The method for producing porous cellulose particles according to any one of [1] to [13] above, the method comprising the following steps:

a step of impregnating the porous cellulose particles with a collagen solution;
a step of neutralizing the collagen solution to pH 6 to 8;
a dewatering step in which the collagen solution is removed; and
a drying step in which the porous cellulose particles are dried.

[ADVANTAGEOUS EFFECTS OF INVENTION]

The porous cellulose particles of the invention, when used as a microcarrier serving as a culture carrier, allow cells to be efficiently adsorbed during the initial stage of culturing when substrate adhesion between the cells is still undeveloped, while also reducing shedding of cells from the particles by the effects of shear stress even after high density culturing, and allowing the cells to be highly efficiently grown.

DESCRIPTION OF EMBODIMENTS

The invention will now be explained in detail by embodiments.

One embodiment of the invention is porous cellulose particles composed of cellulose, which are porous cellulose particles having collagen on the porous cellulose particle surfaces. The porous cellulose particles of the embodiment have a fixed amount of collagen in dry state of 10 $\mu$g/g to 25 mg/g (0.001 to 2.5 wt%) per 1 g of porous cellulose particles.

Throughout the present specification, the term "surfaces" in the term "porous cellulose particle surfaces" encompasses not only the outer surfaces of the porous cellulose particles but also the surfaces of the voids inside the particles.

The type of cellulose composing the porous cellulose particles for this embodiment is not particularly restricted, and any publicly known cellulose such as copper-ammonia regenerated cellulose, viscose regenerated cellulose, cotton, pulp or polynosic may be used, among which copper-ammonia regenerated cellulose and viscose regenerated cellulose are preferred, and copper-ammonia regenerated cellulose is more preferred. Copper-ammonia regenerated cellulose has a low degree of crystallinity and is therefore highly reactive during functional group modification, such that even with porous particles it is possible to modify the cationic substituents uniformly at high density throughout the particle interiors. Copper-ammonia regenerated cellulose also has the feature of lower zeta-potential compared to common crystalline cellulose. It is therefore unlikely for interaction such as electrostatic adsorption or repulsion to be produced between collagen molecules, allowing the collagen to be homogeneously introduced into the porous inner surfaces.

The lower limit for the density of the material used for the microcarriers is preferably 1.0 g/cm$^3$ or greater. It is more preferably 1.2 g/cm$^3$ or greater and particularly preferably 1.4 g/cm$^3$ or greater. The upper limit for the density of the material used for the microcarriers is preferably lower than 2.0 g/cm$^3$. It is more preferably 1.8 g/cm$^3$ or lower and particularly preferably 1.6 g/cm$^3$ or lower. If the density is lower than 2.0 g/cm$^3$, it will be possible to produce a suspension even with low stirring force, thus reducing shear stress and minimizing damage to cells. If the density is 1.0 g/cm$^3$ or

greater, the microcarriers will sink down into the water preventing the cells from contacting with air and thus allowing them to be used for agitation culture. The cellulose density is preferably 1.5 g/cm$^3$, as the density for a material to be used as a microcarrier.

The form of the porous cellulose particles of the embodiment is preferably spherical, and more preferably true spherical with true sphericity of 0.70 to 1.00, with true sphericity of 0.90 to 1.00 being even more preferred. As used herein, the term "sphericity" is defined as the value representing the shapes of the particles, when the porous particles have been swelled with water and the excess water subsequently removed, and the shortest diameter among projection circles on the particles in an optical microscope image is divided by the longest diameter. As a specific swelling step for the porous particles, 1 mg of porous particles was immersed in 10 ml of physiological saline for half a day for until the particles swelled, and then an empty column (7311550 by Bio-RAD Co.) was used for filtration and removal of the excess water until the particles fell under their own weight. Although this measuring method only considers the particle on one plane, using the average value for measurement of at least 100 or more particles makes it possible to account for variation in different observation directions, allowing the three-dimensional sphericity of the particles to be determined. A value closer to 1.00 indicates a more spherical particle shape, with 1.00 representing total sphericity.

Since a spherical porous microcarrier has no anisotropy in terms of interior liquid permeability, collagen can be uniformly modified throughout the particle interiors, allowing the cells to infiltrate in a uniform manner throughout the particle interiors. It is also possible to obtain buoyancy with lower stirring force compared to other shapes such as cubes, lowering the shear stress and helping to reduce shedding.

The lower limit for the mean particle size of the porous cellulose particles of the embodiment is preferably 100 $\mu$m or greater and more preferably 200 $\mu$m or greater. A mean particle size of greater than 100 $\mu$m will result in more cells being held per microcarrier, thus facilitating separation from the medium. The upper limit for the mean particle size of the porous cellulose particles of the embodiment is preferably 1000 $\mu$m or smaller, more preferably 500 $\mu$m or smaller and even more preferably 400 $\mu$m or smaller. A mean particle size of smaller than 1000 $\mu$m will allow nutrients and oxygen to be sufficiently supplied to the cells at the center sections of the microcarrier, resulting in a satisfactory culturing environment. A mean particle size of smaller than 1000 $\mu$m will also require less stirring force to suspend and disperse the microcarrier, thus lowering the shear stress. Shedding of cells will thus be less likely to occur, making the method more suitable for high-density culture. The term "mean particle size" as used herein is the average particle diameter measured for 20 particles or more, after removing the excess water once the porous particles have been swelled with water, and setting an appropriate magnification with an optical microscope. As a specific swelling step for the porous particles, 1 mg of porous particles was immersed in 10 ml of physiological saline for half a day until the particles swelled, and then an empty column (7311550 by Bio-RAD Co.) was used for filtration and removal of the excess water until the particles fell under their own weight.

The lower limit for the mean opening size of the voids of the porous cellulose particles of the embodiment is a mean opening size of preferably 5 $\mu$m or greater and more preferably 10 $\mu$m or greater. If the mean opening size is 5 $\mu$m or greater, the degree of freedom of movement of the cells or culture solution in the microcarrier will increase, and cells will be able to infiltrate into the microcarrier interiors, allowing efficient proliferation of the cells. The upper limit for the mean opening size is preferably 100 $\mu$m or smaller, more preferably 80 $\mu$m or smaller and even more preferably 50 $\mu$m or smaller. A mean opening size of 100 $\mu$m or smaller can ensure sufficient surface area for cell growth, for efficient proliferation of the cells. The porous cellulose particles of the embodiment preferably form a continuous pore structure with adjacent voids mutually communicating by openings in the membranes dividing them. When a continuous pore structure is formed, the specific surface area increases and the cells can also infiltrate throughout the microcarrier, thus increasing the number of adhering cells.

As used herein, the term "mean opening size" refers to the maximum value of the pore distribution determined by replacing the porous particle solvent in the order: water, ethanol, t-butanol, and then removing the residual excess solvent by freeze-drying, and measuring using a mercury intrusion porosimeter.

The lower limit for the surface area (specific surface area) of the porous cellulose particles of the embodiment per 1 g of weight during drying is preferably 0.3 m$^2$/g or greater, more preferably 0.6 m$^2$/g or greater and even more preferably 0.8 m$^2$/g or greater. The surface area necessary for cell culturing is preferably 0.3 m$^2$/g or greater from the viewpoint of causing efficient proliferation of the cells, and also from the viewpoint of reducing the amount of collagen per unit area so as to help prevent adhesion between the particles by anchoring of the collagen molecules. The upper limit for the specific surface area is preferably 4.4 m$^2$/g or lower, more preferably 3.3 m$^2$/g or lower, even more preferably 2.0 m$^2$/g or lower and particularly preferably 1.7 m$^2$/g or lower. The specific surface area is preferably 4.4 m$^2$/g or lower from the viewpoint of not lowering the charge per unit area or the amount of collagen or lowering the cell adhesion rate, and also from the viewpoint of preventing shedding by the effects of shear stress when culturing at high density.

The term "specific surface area" used herein is the surface area per 1 g of dry weight as measured by the BET method.

The cellulose composing the porous cellulose particles described herein is preferably modified with a cationic substituent. The cationic substituent is preferably at least one type selected from the group consisting of primary amino, secondary amino and tertiary amino groups, more preferably a tertiary amino group, and even more preferably a diethylaminoethyl group.

For the charge capacity of the porous cellulose particles of the embodiment, unlike particles of polystyrene or gelatin, it is also necessary to take into account the effects of hydroxyl groups in the cellulose substrate, and the fact that hydrogen bonding and the degree of crystallinity inside the molecules differs significantly compared to particles of polysaccharides, such as dextran particles. It is also important to consider that collagen-fixed cationized cellulose and non-collagen-fixed cationized cellulose have different optimal charge ranges for satisfactory adhesion of the cells. The upper limit for the charge capacity of the porous cellulose particles of the embodiment is preferably 5.0 mmol/g or lower, more preferably 3.0 mmol/g or lower and particularly preferably 2.0 mmol/g or lower. If the charge capacity is lower than 5.0 mmol/g, proliferation of the cells will be less likely to be inhibited. The lower limit for the charge capacity of the porous cellulose particles of the embodiment is preferably 0 mmol/g or higher, more preferably higher than 0 mmol/g, more preferably 0.005 mmol/g or higher, more preferably 0.1 mmol/g or higher, more preferably 0.3 mmol/g or higher and particularly preferably 0.5 mmol/g or higher. If the lower limit for the charge capacity of the porous cellulose particles is within this range it will be possible to efficiently adsorb cells onto the particles. As used herein, the term "charge capacity" refers to the charge capacity determined by evaluation using 1 N silver nitrate (with potassium chromate as the indicator) for titration of the chloride ion concentration of a solution obtained by adsorbing chloride ion onto an amine in the porous particles using hydrochloric acid and then washing off the chloride ion adsorbed by a sodium sulfate solution, and after evaluation determining the mean value measured for 3 or more measured samples. More specifically, the charge capacity can be calculated by the following measuring method.

[Measurement of charge capacity]

The porous particles are measured out to 5.0 g and a funnel and suction filter are used for washing twice with 30 mL of distilled water, for exchange with water. The sample is then washed twice with 30 mL of a 0.3 N aqueous hydrochloric acid solution and 4 times with 50 mL of a $10^{-4}$ N HCl aqueous hydrochloric acid solution, in that order. A funnel and suction filter are then used for washing 4 times with 50 mL of a 10% aqueous sodium sulfate solution, and the filtrate is collected. A 2 mL portion of a 0. 1 N potassium chromate aqueous solution is added to the collected filtrate and the mixture is stirred. Titration is performed with a 1 N aqueous silver nitrate solution, and the titer A (mL) is determined at the moment that reddish-brown precipitation of silver chromate begins to appear.

The sample that has completed washing with the 10% aqueous sodium sulfate solution in the previous step is separately washed 4 times with 50 mL of purified water, and then dried at 105°C for 15 hours or longer, after which the weight is measured with a balance to determine the dry weight B (g).

For measurement of the charge capacity, the measured titer A (mL) and the dry weight B (g) of the sample are used for calculation according to the following formula (1) (N = 3).

$$\text{Charge capacity (mmol/g)} = 1(N)\ (\text{mol/L}) \times A\ (\text{mL})/B\ (\text{g}): \text{Formula (1)}$$

The fixed amount of the collagen is preferably 10 μg/g to 25 mg/g (0.001 to 2.5 wt%) per 1 g of cell culture carrier (dry porous cellulose particles).

The lower limit for the fixed amount of the collagen is preferably 10 μg/g or greater, more preferably 100 μg/g or greater, more preferably 500 μg/g or greater, more preferably 1 mg/g or greater and particularly preferably 2 mg/g or greater, per 1 g of cell culture carrier (dry porous cellulose particles). In terms of the fixed amount per unit area of the culture carrier, the lower limit is preferably 1 ng/cm$^2$ or greater, more preferably 10 ng/cm$^2$ or greater, more preferably 50 ng/cm$^2$ or greater, more preferably 100 ng/cm$^2$ or greater and particularly preferably 200 ng/cm$^2$ or greater. The upper limit is preferably 25 mg/g or less, more preferably 20 mg/g or less, more preferably 15 mg/g or less and particularly preferably 10 mg/g. In terms of the fixed amount per unit area of the culture carrier, the amount is preferably 2.5 μg/cm$^2$ or less, more preferably 2 μg/cm$^2$ or less, more preferably 1.51 μg/cm$^2$ or less and particularly preferably 1 μg/cm$^2$ or less. If the lower limit is within this range it will be possible to improve the cell adhesion and infiltration, and an upper limit within this range will avoid dispersion defects caused by filling of the cellulose particle pores with the fiberized collagen molecules or adhesion between particles by anchoring of the collagen molecules. The amount of collagen for this embodiment was quantified by hydroxyproline assay. More specifically, the collagen amount can be calculated by the following measuring method.

[Measurement of collagen amount]

The collagen amount for this embodiment may be quantified using a hydroxyproline assay kit (Total Collagen Assay Kit by QuickZyme).

A 10 mg portion of collagen-coated sample is weighed out, 1 ml of PBS buffer is added, and the mixture is stirred for 7 days using a rotator at a stirring speed of 20 rpm. After 7 days, a spin column (EP-31101 by Bio-Pharma) is used for filtration and centrifugal separation for 1 minute with a 2000 G small centrifuge (CHIBITAN-R, CT6M by Millipore), to separate the particles and supernatant. After removing the separated supernatant, 1 ml of PBS is added to remove the supernatant remaining on the particles, and centrifugal separation is performed for 1 minute with a 2000 G small centrifuge (CHIBITAN-R, CT6M by Millipore), recovering the washed particles. When the particles and supernatant are separated, the collagen eluted into the supernatant is removed and the amount of collagen fixed on the particles can be measured. A 500 $\mu$l portion of 6 M hydrochloric acid is added to the particles, and the mixture is heated at 95°C for 24 hours for hydrolysis of the collagen to produce hydroxyproline. After heating, 120 $\mu$l of 48% sodium hydroxide and 130 $\mu$l of purified water are added to 500 $\mu$l of sample for neutralization. The neutralized sample is passed through a PES syringe filter with a basis weight of 0.2 $\mu$l, and the fine particles remaining without hydrolysis are removed.

For drawing of a calibration curve, a 500 $\mu$l portion of a 1000 $\mu$g/ml collagen solution is weighed out, 500 $\mu$l of 12 M hydrochloric acid is added, and the mixture is heated at 95°C for 24 hours. A 1 ml portion of the calibration curve sample is neutralized with 240 $\mu$l of 48% sodium hydroxide and 260 $\mu$l of purified water. The neutralized sample is diluted to prepare 7 samples with collagen concentrations of 0 $\mu$g/ml, 7.8 $\mu$g/ml, 15.6 $\mu$g/ml, 31.3 $\mu$g/ml, 62.5 $\mu$g/ml, 125 $\mu$g/ml and 250 $\mu$g/ml.

The neutralized sample and calibration curve drawing sample are added at 35 $\mu$l each to a 96-well microplate. Next, 70 $\mu$l of a Chloramine-T solution (a mixture obtained by adding 26 ml of 2-propanol to the aforementioned reagent with 1.86 g sodium hydroxide, 2.18 g citric acid monohydrate, 2.75 g anhydrous sodium acetate, 0.5 g glacial acetic acid and 0.84 g of Chloramine-T, adjusted upward to 74 ml with water), is added to each well, and reaction is conducted at room temperature for 20 minutes for conversion to pyrrole. After then adding 70 $\mu$l of Ehrlich's reagent to each well, reaction is conducted at 60°C for 30 minutes. A microplate reader (SH-1000Lab by Corona Co.) is used to measure the absorbance at a wavelength of 560 nm. The intercept t and slope k are calculated from the calibration curve with collagen concentration on the abscissa and absorbance on the ordinate, and the collagen concentration C of the sample solution is calculated from the absorbance A of each sample, using the following formula:

$$C\ [\mu g/ml] = (A - t)/k.$$

When the absorbance A is obtained by extrapolation from the calibration curve, the sample solution may be appropriately diluted for remeasurement of the absorbance, compensating the collagen concentration with an increased dilution factor. The amount of sample used is recorded as M [mg], and the fixed amount of collagen (amount of shedding) is determined by the following formula.

Fixed amount of collagen (mg/g) = C $[\mu g/ml] \times$ (dilution ratio during neutralization: 1.5 times) $\times$ (pre-neutralization sample amount: 0.5 ml)/M [mg]

The collagen can be introduced by physical adsorption or chemical bonding. More specifically, it may be introduced into the cellulose surface by several methods, such as a method of impregnating the cellulose with a collagen solution and adsorbing it by interaction between the cellulose and collagen in the solution, a method of impregnating the collagen and then neutralizing it with a buffer and drying it, to deposit and fix the collagen fibers on the cellulose surfaces, or a method of modification by chemical bonding such as condensation of primary amino groups and carboxylic acid. There are no particular restrictions on the introduction method for this embodiment, but it is preferably a method in which the collagen is dried after neutralization and the collagen is deposited and fixed on the cellulose surface. Neutralization of collagen with a buffer near pH 7 causes the collagen to fiberize, and subsequent drying treatment can firmly bond the collagen onto the cellulose surfaces. If the collagen is fixed on the cellulose by this method, the collagen will be held without detachment for prolonged periods even when liquid is added to the particles and the mixture is stirred. In addition, since a large amount of collagen can be introduced into the particles, cells are able to satisfactorily adhere.

After being introduced into the cellulose particles, the collagen may be chemically crosslinked with a crosslinking agent. There are no particular restrictions on the type of crosslinking agent, and crosslinking may be with a crosslinking agent such as glutaraldehyde, formaldehyde, 1-[3-(dimethylamino)propyl]-3-ethylcarbodiimide, N,N'-dicyclohexylcarbodiimide, N,N'-diisopropylcarbodiimide, N,N'-carbonyldiimidazole, 1,1'-carbonyldi(1,2,4-triazole), 4-(4,6-dimethoxy-1,3,5-triazin-2-yl)-4-methylmorpholinium chloride hydrate, (4,6-dimethoxy trifluoromethanesulfonate-1,3,5-triazin-2-yl)-(2-octoxy-2-oxoethyl)dimethylammonium, 1H-benzotriazol-1-yloxytris(dimethylamino)phosphonium hexafluorophosphate, 1H-benzotriazol-1-yloxytripyrrolidinophosphonium hexafluorophosphate, O-(benzotriazole-1-yl)-N,N,N',N'-tetramethyluronium hexafluorophosphate or O-(7-azabenzotriazole-1-yl)-N,N,N',N'-tetramethyluronium hexafluorophosphate. A carboxylic acid activating reagent such as N-hydroxysuccinic acid imide may also be added in addition to the crosslinking agent. Crosslinking of collagen will strengthen adhesion onto the cellulose, allowing a cell anchoring effect to be maintained even during agitation culture.

The porous cellulose particles of the embodiment can be produced by a method of molding a solution of cellulose into a desired shape while cooling it to below the solidification temperature for freezing, and then either removing the solvent by extraction or inactivating its dissolution power. The freezing temperature is generally preferred to be set no lower than 40°C below the freezing temperature of the solvent, and will usually be selected in a range of 0 to 20°C lower than the freezing temperature.

The frozen cellulose solution or cellulose derivative solution then has the cellulose- or cellulose derivative-dissolving solvent removed by extraction, or else its solubility is lowered (both of these treatments may also be collectively referred to herein as "solvent removal"), to obtain a solidified cellulose porous body. The conditions for solvent removal are not particularly restricted. It is usually sufficient to rapidly introduce the frozen body into an arbitrary coagulating bath or regenerating bath, with the coagulating bath or regenerating bath preferably being at no higher than the freezing temperature of the solution. A cellulose derivative, however, requires a cellulose regeneration step, where the regeneration is carried out simultaneously with or successively with (i.e. after) the solvent removal. The regeneration itself may be carried out by a common method. Using the production method described above does not require introduction of extraneous materials such as porous materials into the polymer solution during production, and it therefore allows droplets with fine uniform diameters to be easily created, for desired control of the particle sizes. The diameters and shapes of the voids are primarily determined by the sizes and shapes of the solvent crystals formed during freezing solidification of the solvent in the solution. The freezing solidification conditions including the type and temperature of polymer solution may therefore be altered to adjust the shapes and sizes of the voids.

The porous cellulose particles referred to herein may be used directly as granulated cellulose particles, but they are preferably used after crosslinking. The crosslinking method is not restricted, and crosslinking between the cellulose porous body molecules may be accomplished using a crosslinking agent having two or more functional groups that can react and bond with the hydroxyl groups of cellulose. One example is a bifunctional organic substance. An example of such a substance is one of the form X-R-Z [where R represents an aliphatic residue containing a carbon atom, and X and Z are halogens or epoxy groups which are bonded to the carbon atoms of the aliphatic residues], which reacts relatively easily in the presence of an alkaline reactive substance. Examples of bifunctional compounds suitable for reaction include, but are not particularly limited to, epichlorohydrin, dichlorohydrin, 1,2- or 3,4-diepoxybutane, bisepoxypropyl ether, ethylene glycol-bis-epoxy-propyl ether, 1,4-butanediol-bis-epoxypropyl ether, and their closely related compounds. Crosslinking allows the crystal structure of the cellulose to be maintained even with abundant modification with cationic substituents, so that the particle structure can be kept stable.

There are no particular restrictions on the method of modifying the cationic substituents on the cellulose composing the porous cellulose particles in the porous cellulose particles of the embodiment, and introduction of groups may be by multiple methods including methods of direct modification of hydroxyl groups and methods of modification via functional groups with epoxy groups. For introduction of a tertiary amino group, a method of directly modifying the hydroxyl groups of the cellulose using 2-(diethylamino)ethyl chloride hydrochloride is preferred. Because of the high abundance of hydroxyl groups on cellulose, it is possible to achieve uniform modification of numerous cationic tertiary amino groups throughout the interiors of the particles by direct modification of hydroxyl groups.

The porous cellulose particles of the embodiment can be used as a culturing microcarrier. The term "culturing microcarrier" here refers to a microcarrier used for adhesion and proliferation of cells to be cultured. With culturing, the desired product may be the cells themselves, or it may be viruses for vaccine production, as a product of cell growth. In the latter case, cells suitable as hosts may be selected. A preferred embodiment may also be using genetically modified cells having a gene introduced as the desired cell growth product. The culturing may be by suspension culture or stationary culture, for example, with no particular limitations on their use.

The cells to be used for culturing are not particularly restricted and may be bacteria, yeast, plant cells or animal cells, including cells from fish or insects, but they are preferably primate or human stem cells, or induced pluripotent stem cells. Human stem cells include dental pulp stem cells, neural stem cells, skin stem cells, hematopoietic stem cells, mesenchymal stem cells, mammary stem cells, endothelial stem cells and embryonic stem cells, with no particular limitation to these.

EXAMPLES

The present invention will now be explained in more specific detail through the following Examples and Comparative Examples, with the understanding that the invention is in no way limited to the Examples.

The methods for measuring the physical property values of the structure described above will now be explained.

(1) Charge capacity

A 1.0 g portion of a water-containing sample was measured out and washed twice with 30 mL of 0.3 N aqueous hydrochloric acid solution and 4 times with 50 mL of $10^{-4}$ N aqueous hydrochloric acid solution, using a funnel and suction filter. A funnel and suction filter were then used for washing 4 times with 50 mL of a 10% aqueous sodium sulfate solution, and the filtrate was collected. A 0.1 N aqueous potassium chromate solution was added 2 mL each time to the collected filtrate and the mixture was stirred. Titration was performed with a 1 N silver nitrate aqueous solution, and the titer A (mL) was determined at the moment that reddish-brown precipitation of silver chromate began to appear.

The sample that had completed washing with the 10% aqueous sodium sulfate solution in the previous step was separately washed 4 times with 50 mL of purified water, and then dried at 105°C for 15 hours or longer, after which the weight was measured with a balance to determine the dry weight B (g).

For measurement of the charge capacity, the measured titer A (mL) and the dry weight B (g) of the sample were used for calculation by the following formula (1) (N = 3).

$$\text{Charge capacity (mmol/g)} = 1(\text{N}) \text{ (mol/L)} \times \text{A (mL)/B (g)}: \text{Formula (1)}$$

(2) Measurement of collagen amount

The collagen amount for this embodiment was quantified using a hydroxyproline assay kit (Total Collagen Assay Kit by QuickZyme).

A 10 mg portion of collagen-coated sample was weighed out, 1 ml of PBS buffer was added, and the mixture was stirred for 7 days using a rotator at a stirring speed of 20 rpm. After 7 days, a spin column (EP-31101 by Bio-Pharma) was used for filtration and centrifugal separation for 1 minute with a 2000 G small centrifuge (CHIBITAN-R, CT6M by Millipore), to separate the particles and supernatant. After removing the separated supernatant, 1 ml of PBS was added to remove the supernatant remaining on the particles, and centrifugal separation was performed for 1 minute with a 2000 G small centrifuge (CHIBITAN-R, CT6M by Millipore), recovering the washed particles. When the particles and supernatant are separated, the collagen eluted into the supernatant is removed and the amount of collagen fixed on the particles can be measured. A 500 $\mu$l portion of 6 M hydrochloric acid was added to the particles, and the mixture was heated at 95°C for 24 hours for hydrolysis of the collagen to produce hydroxyproline. After heating, 120 $\mu$l of 48% sodium hydroxide and 130 $\mu$l of purified water were added to 500 $\mu$l of sample for neutralization. The neutralized sample was passed through a PES syringe filter with a basis weight of 0.2 $\mu$l, and the fine particles remaining without hydrolysis were removed.

For drawing of a calibration curve, a 500 $\mu$l portion of a 1000 $\mu$g/ml collagen solution was weighed out, 500 $\mu$l of 12 M hydrochloric acid was added, and the mixture was heated at 95°C for 24 hours. A 1 ml portion of the calibration curve sample was neutralized with 240 $\mu$l of 48% sodium hydroxide and 260 $\mu$l of purified water. The neutralized sample was diluted to prepare 7 samples with collagen concentrations of 0 $\mu$g/ml, 7.8 $\mu$g/ml, 15.6 $\mu$g/ml, 31.3 $\mu$g/ml, 62.5 $\mu$g/ml, 125 $\mu$g/ml and 250 $\mu$g/ml.

The neutralized sample and calibration curve drawing sample were added at 35 $\mu$l each to a 96-well microplate. Next, 70 $\mu$l of a Chloramine-T solution (a mixture obtained by adding 26 ml of 2-propanol to the aforementioned reagent with 1.86 g sodium hydroxide, 2.18 g citric acid monohydrate, 2.75 g anhydrous sodium acetate, 0.5 g glacial acetic acid and 0.84 g of Chloramine-T, adjusted upward to 74 ml with water), was added to each well, and reaction was conducted at room temperature for 20 minutes for conversion to pyrrole. After then adding 70 $\mu$l of Ehrlich's reagent to each well, reaction was conducted at 60°C for 30 minutes. A microplate reader (SH-1000Lab by Corona Co.) was used to measure the absorbance at a wavelength of 560 nm. The intercept t and slope k were calculated from the calibration curve with collagen concentration on the abscissa and absorbance on the ordinate, and the collagen concentration C of the sample solution was calculated from the absorbance A of each sample, using the following formula:

$$\text{C } [\mu\text{g/ml}] = (\text{A - t})/\text{k}.$$

When the absorbance A is obtained by extrapolation from the calibration curve, the sample solution may be appropriately diluted for remeasurement of the absorbance, compensating the collagen concentration with an increased dilution factor. The amount of sample used was recorded as M [mg], and the amount of fixed collagen (amount of shedding) was determined by the following formula.

Fixed amount of collagen (mg/g) = C [$\mu$g/ml] $\times$ (dilution ratio during neutralization: 1.5 times) $\times$ (pre-neutralization sample amount: 0.5 ml)/M [mg]

A method for producing the porous cellulose particles will now be described.

[Preparation of cellulose particles]

A cellulose cuprammonium solution with a viscosity of 2 poise at 5°C, a cellulose concentration of 2%, a copper concentration of 1.2% and an ammonia concentration of 4%, prepared using refined linter as the starting material, was sprayed in an amount of 500 mL together with nitrogen gas using a two-fluid nozzle, and was used while being stirred in 10 L of silicon oil (SH200, 1.5 cs, Toray Co., Ltd.) that had been cooled to -40°C. The silicon oil was heated to -20°C while continuing stirring for 20 minutes, and then 10 L of 40% sulfuric acid that had been cooled to -35°C was loaded in. After loading the sulfuric acid, stirring was continued for 8 hours, the temperature was raised to 20°C, and the silicon oil was removed, extracting and washing off the remainder, to obtain porous cellulose particles. A classifying sieve (mesh opening: 250 μm · 125 μm) was used to obtain porous cellulose particles having a mean particle size of 240 μm and a mean opening size of 30 μm. Next, 50 g of the obtained particles was measured out for epichlorohydrin crosslinking, after which 2010 ml of purified water, 0.12 mg of sodium dodecyl sulfate, 97.9 ml of a 48% sodium hydroxide aqueous solution and 28.9 g of epichlorohydrin were added and reaction was conducted while stirring for 1 hour in a thermostatic bath at 60°C. A funnel and suction filter were then used for washing of the sample 3 times with 8000 mL of purified water, and the cellulose particles before cationization modification were used as "particle 1".

[Cationization of cellulose particles]

A 6.66 g portion of particle 1 was measured out, after which purified water, anhydrous sodium sulfate, sodium dodecyl sulfate and an aqueous solution of 2-(diethylamino)ethyl chloride hydrochloride (50%) were added in the amounts listed in Table 1 below, and the mixture was stirred. A reaction starting solution (48% sodium hydroxide aqueous solution) was immediately added, and reaction was conducted while stirring for 1 hour in a thermostatic bath at 70°C. After an elapse of 1 hour, 36% hydrochloric acid was added to pH 7 for neutralization and the sample was washed 3 times by suction filtration with 8000 mL of purified water to obtain cationic porous cellulose particles, as particles 2 to 8. The charge capacity of the dried particles was measured.

[Table 1]

|  | Particle 2 | Particle 3 | Particle 4 | Particle 5 | Particle 6 | Particle 7 | Particle 8 |
|---|---|---|---|---|---|---|---|
| Purified water | 217.3 g | 217.3 g | 236.5 g | 236.5 g | 218.3 g | 218.3 g | 218.3 g |
| Anhydrous sodium sulfate | 88.8 g | 88.8 g | 91.5 g | 93.2 g | 89.3 g | 124.3 g | 146.7 g |
| Sodium dodecyl sulfate | 0.016 g | 0.016 g | 0.016 g | 0.016 g | 0.016 g | 0.016 g | 0.016 g |
| 2-(Diethylamino)ethyl chloride hydrochloride aqueous solution | 0.0012 g | 0.46 g | 2.32 g | 4.65 g | 11.1 g | 27.8 g | 44.4 g |
| Sodium hydroxide | 0.0002 g | 0.39 g | 1.94 g | 3.87 g | 5.36 g | 13.4 g | 21.4 g |
| Charge capacity of particles obtained after reaction [mmol/g] | 0.005 | 0.1 | 0.5 | 0.9 | 1.8 | 2.5 | 3.0 |

[Collagen coating by dry method]

Next, 5 ml of a pH 3.0 collagen solution with the concentration as listed in Table 2 was added to particle groups 1 to 8 (100 mg), and the mixture was incubated for 1 hour at 30°C. After incubation, 5 ml of PBS at pH 7.4 was added and the mixture was mixed by inversion and neutralized. The pH after neutralization was confirmed to be approximately 7.0. An empty column (7311550, product of Bio-RAD) was used to filter off the reaction solution until it fell under its own weight. The filtered particles were dried at 30°C for 2 days. To the obtained dried particles there was added a reaction solution (5 ml of MES buffer adjusted to pH 5.5, 5 mg of EDCI·HCl, 1.15 mg of NHS), and reaction was conducted for 1 hour at 37°C. After the reaction, an empty column (7311550 by Bio-RAD) was used for one minute of centrifugal filtration at 2000 rpm, 5 ml of PBS was added, and the step of centrifugal filtration was repeated 5 times for washing. The washed particles were transferred to a tray, 6 ml of PBS was added and the mixture was dried at 30°C to obtain collagen-modified particles 9 to 26. The amount of collagen in the dried particles was evaluated by hydroxyproline assay.

[Table 2]

| Sample name | Particle 9 | Particle 10 | Particle 11 | Particle 12 | Particle 13 | Particle 14 | Particle 15 | Particle 16 | Particle 17 | Particle 18 | Particle 19 | Particle 20 | Particle 21 | Particle 22 | Particle 23 | Particle 24 | Particle 25 | Particle 26 |
|---|---|---|---|---|---|---|---|---|---|---|---|---|---|---|---|---|---|---|
| Particles used in reaction | Particle 1 | Particle 2 | Particle 3 | Particle 4 | Particle 5 | Particle 6 | Particle 7 | Particle 8 | Particle 1 | Particle 1 | Particle 1 | Particle 6 | Particle 6 | Particle 6 | Particle 6 | Particle 6 | Particle 6 | Particle 6 |
| Charge capacity of particles [mmol/g] | 0 | 0.005 | 0.1 | 0.5 | 0.9 | 1.8 | 2.5 | 3.0 | 0 | 0 | 0 | 1.8 | 1.8 | 1.8 | 1.8 | 1.8 | 1.8 | 1.8 |
| Collagen solution reaction concentration [mg/ml] | 0.5 | 0.5 | 0.5 | 0.5 | 0.5 | 0.5 | 0.5 | 0.5 | 3 | 0.05 | 0.0005 | 8 | 6.3 | 5 | 3 | 0.05 | 0.0005 | 9.5 |
| Fixed Amount of collagen to particles [mg/g] | 3.8 | 3.8 | 3.7 | 3.6 | 3.5 | 1.8 | 2.3 | 1.9 | 10.1 | 0.2 | 0.01 | 25.2 | 20.1 | 15.2 | 10.1 | 0.2 | 0.01 | 29.9 |

[Collagen coating by wet method]

After adding 5 ml of a 0.5 mg/ml concentration collagen solution to particle 1 (100 mg), the mixture was incubated at 30°C for 1 hour. After incubation, 5 ml of PBS was added, and the mixture was mixed by inversion and filtered using an empty column (7311550 by Bio-RAD) until it fell under its own weight. A 5 ml portion of PBS was passed through the column 3 times for washing to obtain particle 27. This method was employed as the wet method. When the fixed amount of collagen of particle 27 was evaluated, the fixed amount was found to be 0.1 mg/g. When the same particle 1 was used in the dry method, the amount of collagen was markedly reduced compared to the fixed amount of 3.8 mg/g for particle 9 which had been prepared to the same collagen concentration.

[Crosslinked collagen-coated particles using glutaraldehyde]

After adding 5 ml of a 0.5 mg/ml concentration collagen solution to particle 6 (100 mg), the mixture was incubated at 30°C for 1 hour. After incubation, 5 ml of PBS was added, and the mixture was mixed by inversion and filtered using an empty column (7311550 by Bio-RAD) until it fell under its own weight. The filtered particles were dried at 30°C for 2 days. To the obtained dried particles there was added a reaction solution (5 ml of PBS buffer, 50 $\mu$l of glutaraldehyde), and reaction was conducted for 1 hour at 37°C. After the reaction, an empty column (7311550 by Bio-RAD) was used for centrifugal filtration at 2000 rpm, 5 ml of PBS was added, and the step of centrifugal filtration was repeated 5 times for washing. The washed particles were transferred to a tray, 6 ml of PBS was added and the mixture was dried for 2 days at 30°C to obtain collagen-modified particle 28. The amount of collagen in the dried particles was evaluated by hydroxyproline assay, and the fixed amount was found to be 2.0 mg/g.

[Uncrosslinked collagen-coated particles]

After adding 5 ml of a 0.5 mg/ml concentration collagen solution to particle 6 (100 mg), the mixture was incubated at 30°C for 1 hour. After incubation, 5 ml of PBS was added, and the mixture was mixed by inversion and filtered using an empty column (7311550 by Bio-RAD) until it fell under its own weight. The filtered particles were dried at 30°C for 2 days to obtain particle 29. The amount of collagen in the dried particles was evaluated by hydroxyproline assay, and the fixed amount was found to be 2.0 mg/g.

[Collagen-coated cellulose particles with mean opening size of 1 $\mu$m]

A cellulose cuprammonium solution with a viscosity of 2 poise at 5°C, a cellulose concentration of 2%, a copper concentration of 1.2% and an ammonia concentration of 4%, prepared using refined linter as the starting material, was sprayed in an amount of 500 mL together with nitrogen gas using a two-fluid nozzle, and was used while being stirred in 10 L of silicon oil (SH200, 1.5 cs, Toray Co., Ltd.) that had been cooled to -55°C. The silicon oil was kept at -55°C while continuing stirring for 50 minutes, and then 10 L of 40% sulfuric acid that had been cooled to -55°C was loaded in. After loading the sulfuric acid, stirring was continued for 8 hours, the temperature was raised to 20°C, and the silicon oil was removed, extracting and washing off the remainder, to obtain porous cellulose particles. A classifying sieve (mesh opening: 250 $\mu$m · 125 $\mu$m) was used to obtain porous cellulose particles having a mean particle size of 240 $\mu$m and a mean opening size of 1 $\mu$m. After then adding 5 ml of a 0.5 mg/ml concentration collagen solution to the obtained particles, the mixture was incubated at 30°C for 1 hour. After incubation, 5 ml of PBS was added, and the mixture was mixed by inversion and filtered using an empty column (7311550 by Bio-RAD) until it fell under its own weight. The filtered particles were dried at 30°C for 2 days. To the obtained dried particles there was added a reaction solution (5 ml of MES buffer adjusted to pH 5.5, 5 mg of EDCI·HCl, 1.15 mg of NHS), and reaction was conducted for 1 hour at 37°C. After the reaction, an empty column was used for centrifugal filtration at 2000 rpm, 5 ml of PBS was added, and the step of centrifugal filtration was repeated 5 times for washing. The washed particles were transferred to a tray, 6 ml of PBS was added and the mixture was dried for 2 days at 30°C to obtain collagen-modified particle 30. The amount of collagen in the dried particles was evaluated by hydroxyproline assay, and the fixed amount was found to be 2.0 mg/g.

Evaluation of cell culturing with the collagen porous particles obtained as described above will now be described.

[Evaluation of culturing]

Table 3-1 and Table 3-2 below show the results of evaluating culturing using particles 1, 6 and 9 to 30.

The cells used for evaluation were human dental pulp-derived stem cells (DPSC) for Examples 1 to 17 and 24 to 27, and Comparative Examples 1 to 3, and human adipocyte-derived mesenchymal stem cells (MSC) for Examples 18 to 23.

[Stationary culture method for human dental pulp-derived stem cells (DPSC)]

Using a 6-well plate for suspension culture by Iwaki (Product No.: 1810-006), $5.0 \times 10^4$ cells derived from human dental pulp-derived stem cells (PT-5025) by Lonza, KK., 3 ml of DPSC BulletKit growth medium (PT-3005) by Lonza, KK. and 6 mg of microcarrier were added to each well, and stationary culturing was continued for 6 days in an incubator at 37°C. The growth medium was exchanged on the 4th day of culturing.

[Method of stationary culturing of human adipocyte-derived mesenchymal stem cells (MSC)]

Using a 6-well plate for suspension culture by Iwaki (Product No.: 1810-006), $5.0 \times 10^4$ human adipose tissue-derived mesenchymal stem cells (Product No.: C-12977) by Promocell, 3 ml of mesenchymal stem cell growth medium 2 (Product No.: C-28009) by Promocell and 6 mg of microcarriers were added to each well, and stationary culturing was carried out for 6 days in an incubator at 37°C. The growth medium was exchanged on the 4th day of culturing.

<Evaluation of cell proliferation using fluorescent microscope>

A step of removing the supernatant from 6 mg of the cultured microcarriers and adding 2 ml of PBS buffer was repeated 3 times, and followed by washing. Calcein-added assay buffer (Live or Dead™ Cell Viability Assay Kit by AAT Bioquest) was added to the washed microcarriers, and the mixture was allowed to stand for 5 minutes. After standing, a step of adding 2 ml of PBS buffer and removing the supernatant was repeated 3 times prior to washing. A fluorescent microscope (DMi8 by Leica) was used to observe viable cells using a 4x magnification lens through an FITC filter (excitation: 480 nm, absorption: 527 nm). Analysis software (Image J) was used to calculate the fluorescence area ratio per particle area, and the initial adhesion and proliferative ability of the cells was evaluated.

<Evaluation of cell proliferation>

The fluorescence area ratio on day 6 with respect to the fluorescence area ratio on day 1 of culturing was evaluated as "5" for an area increase ratio of 800% or greater, "4" for an area increase ratio of less than 800% and 600% or greater, "3" for an area increase ratio of less than 600% and 400% or greater, "2" for an area increase ratio of less than 400% and 300% or greater, and "1" for an area increase ratio of less than 300%.

<Evaluation of initial adhesion>

The initial adhesion of the cells was evaluated as "1" when the ratio of the size of the fluorescence area of the target sample with respect to the fluorescence area on day 1 in Comparative Example 1 was less than 100%, "2" when the area ratio was 100% or greater and less than 200%, "3" when the area ratio was 200% or greater and less than 300%, "4" when it was 300% or greater and less than 400%, or "5" when it was 400% or greater.

<Evaluation of cell adhesion inside carrier>

After fixing the cells onto the particles with 2.5% glutaraldehyde, they were replaced with ethanol and t-butanol and freeze-dried. After coating with osmium, the particles were split with an ion beam and the cross-section was observed with an SEM. 20 particles or more were observed, and evaluated as "1" when the average number of cells per cross-section of each particle was less than 0.5, "2" when it was 0.5 to 1, "3" when it was 2 to 5, "4" when it was 6 to 10 and "5" when it was 11 or more.

<Evaluation of particle aggregation>

The evaluation assigned was "5" when, after adding 1 ml of PBS to 10 mg of particles in a 2 ml tube, each of the particles separated merely by inverted mixing 10 times, "4" when each of the particles separated by 10 pipetting operations using a 1 ml micropipette (3123000063 by Eppendorf), "3" when some of the particles separated by 10 pipetting operations, "2" when none of the particles separated even after 10 pipetting operations, and "1" when none of the particles separated even after 30 pipetting operations followed by 10 minutes in a vortex mixer (VORTEX-GENIE2 by Scientific Industries) with an oscillation level of 6.

<Overall score evaluation>

The total number of points for cell proliferation, initial adhesion, cell adhesion into the carrier and particle aggregation was evaluated as the overall score.

As shown in the following tables, it was confirmed, for example, that in Examples 1 to 27, excellent results were obtained for the evaluation parameters of cell proliferation, cell adhesion into the carrier and/or overall score, compared to Comparative Examples 1 to 3.

In Examples 1 to 7, for example, it was confirmed that excellent results were obtained for the evaluation parameter of overall score, compared to Comparative Example 3 which had the same conditions.

In Examples 8 to 11, it was confirmed that excellent results were obtained for the evaluation parameter of overall score compared to Comparative Example 2 which had the same conditions.

[Table 3-1]

| | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 | Ex. 14 | Ex. 15 | Ex. 16 |
|---|---|---|---|---|---|---|---|---|---|---|---|---|---|---|---|---|
| Cell type | DPSC | DPSC | DPSC | DPSC | DPSC | DPSC | DPSC | DPSC | DPSC | DPSC | DPSC | DPSC | DPSC | DPSC | DPSC | DPSC |
| Particle used No. | Particle 20 | Particle 21 | Particle 22 | Particle 23 | Particle 14 | Particle 24 | Particle 25 | Particle 17 | Particle 9 | Particle 18 | Particle 19 | Particle 10 | Particle 11 | Particle 12 | Particle 13 | Particle 15 |
| Collagen loading amount | 25.2 mg/g (2.5 wt%) | 20.1 mg/g (2.0 wt%) | 15.2 mg/g (1.5 wt%) | 10.1 mg/g (1.0 wt%) | 1.8 mg/g (0.18 wt%) | 200 μg/g (0.02 wt%) | 10 μg/g (0.001 wt%) | 10.1 mg/g (1.0 wt%) | 3.8 mg/g (0.38 wt%) | 200 μg/g (0.02 wt%) | 10 μg/g (0.001 wt%) | 3.8 mg/g (0.38 wt%) | 3.7 mg/g (0.37 wt%) | 3.6 mg/g (0.36 wt%) | 3.5 mg/g (0.35 wt%) | 2.3 mg/g (0.23 wt%) |
| Crosslinking method | EDCI/ NHS | EDCI/ NHS | EDCI/ NHS | EDCII NHS | EDCII NHS | EDCII NHS | EDCI/ NHS | EDCI/ NHS | EDCI/ NHS | EDCI/ NHS | EDCI/ NHS | EDCI/ NHS | EDCI /NHS | EDCI/ NHS | EDCI/ NHS | EDCI/ NHS |
| Charge capacity [mmol/g] | 1.8 | 1.8 | 1.8 | 1.8 | 1.8 | 1.8 | 1.8 | 0 | 0 | 0 | 0 | 0.005 | 0.1 | 0.5 | 0.9 | 2.5 |
| Mean opening size [μm] | 30 | 30 | 30 | 30 | 30 | 30 | 30 | 30 | 30 | 30 | 30 | 30 | 30 | 30 | 30 | 30 |
| Mean particle size [μm] | 240 | 240 | 240 | 240 | 240 | 240 | 240 | 240 | 240 | 240 | 240 | 240 | 240 | 240 | 240 | 240 |
| Sphericity | 0.95 | 0.95 | 0.95 | 0.95 | 0.95 | 0.95 | 0.95 | 0.95 | 0.95 | 0.95 | 0.95 | 0.95 | 0.95 | 0.95 | 0.95 | 0.95 |
| Specific surface area ($cm^2$/g) | 11,000 | 11,000 | 11,000 | 11,000 | 11,000 | 11,000 | 11,000 | 11,000 | 11,000 | 11,000 | 11,000 | 11,000 | 11,000 | 11,000 | 11,000 | 11,000 |
| Cation species | Tertiary amine | Tertiary amine | Tertiary amine | Tertiary amine | Tertiary amine | Tertiary amine | Tertiary amine | None | None | None | None | Tertiary amine | Tertiary amine | Tertiary amine | Tertiary amine | Tertiary amine |
| Cell proliferation (change in cell count: day 6/day 1) | 3 | 3 | 3 | 4 | 4 | 3 | 2 | 4 | 5 | 3 | 2 | 5 | 5 | 5 | 5 | 2 |
| Initial adhesion (adsorbed cell count on day 1) | 4 | 4 | 4 | 5 | 5 | 3 | 2 | 4 | 4 | 2 | 2 | 4 | 4 | 5 | 5 | 3 |

| | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 | Ex. 14 | Ex. 15 | Ex. 16 |
|---|---|---|---|---|---|---|---|---|---|---|---|---|---|---|---|---|
| Particle aggregation | 2 | 2 | 2 | 3 | 4 | 4 | 4 | 3 | 4 | 4 | 4 | 4 | 4 | 4 | 4 | 4 |
| Cell adhesion inside carrier | 1 | 2 | 2 | 3 | 5 | 5 | 4 | 3 | 4 | 4 | 4 | 4 | 4 | 5 | 5 | 4 |
| Overall score | 10 | 11 | 11 | 15 | 18 | 15 | 12 | 14 | 17 | 13 | 12 | 17 | 17 | 19 | 19 | 13 |

[Table 3-2]

| | Ex. 17 | Ex. 18 | Ex. 19 | Ex. 20 | Ex. 21 | Ex. 22 | Ex. 23 | Ex. 24 | Ex. 25 | Ex. 26 | Ex. 27 | Comp. Ex. 1 | Comp. Ex. 2 | Comp. Ex. 3 |
|---|---|---|---|---|---|---|---|---|---|---|---|---|---|---|
| Cell type | DPSC | MSC | MSC | MSC | MSC | MSC | MSC | DPSC | DPSC | DPSC | DPSC | DPSC | DPSC | DPSC |
| Particle used No. | Particle 16 | Particle 9 | Particle 12 | Particle 13 | Particle 14 | Particle 15 | Particle 16 | Particle 29 | Particle 28 | Particle 27 | Particle 30 | Particle 1 | Particle 6 | Particle 26 |
| Collagen loading amount | 1.9 mg/g (0.19 wt%) | 3.8 mg/g (0.38 wt%) | 3.6 mg/g (0.36 wt%) | 3.5 mg/g (0.35 wt%) | 1.8 mg/g (0.18 wt%) | 2.3 (0.23 wt%) | 1.9 mg/g (0.19 wt%) | 2.0 mg/g (0.20 wt%) | 2.0 (0.20 wt%) | 100 (0.01 wt%) | 2.0 mg/g (0.20 wt%) | 0 mg/g | 0 mg/g | 29.9 mg/g (2.99 wt%) |
| Crosslinking method | EDCI/NHS | EDCI/NHS | EDCI/NHS | EDCI/NHS | EDCI/NHS | EDCI/]NHS | EDCI/NHS | None | Glutaraldehyde | Wet method | EDCI/NHS | None | None | EDCI/NHS |
| Charge capacity [mmol/g] | 3.0 | 0 | 0.5 | 0.9 | 1.8 | 2.5 | 3.0 | 1.8 | 1.8 | 0 | 0 | 0 | 1.8 | 1.8 |
| Mean opening size [$\mu$m] | 30 | 30 | 30 | 30 | 30 | 30 | 30 | 30 | 30 | 30 | 1 | 30 | 30 | 30 |
| Mean particle size [$\mu$m] | 240 | 240 | 240 | 240 | 240 | 240 | 240 | 240 | 240 | 240 | 240 | 240 | 240 | 240 |
| Sphericity | 0.95 | 0.95 | 0.95 | 0.95 | 0.95 | 0.95 | 0.95 | 0.95 | 0.95 | 0.95 | 0.95 | 0.95 | 0.95 | 0.95 |
| Specific surface area (cm$^2$/g) | 11,000 | 11,000 | 11,000 | 11,000 | 11,000 | 11,000 | 11,000 | 11,000 | 11,000 | 11,000 | 62,000 | 11,000 | 11,000 | 11,000 |
| Cation species | Tertiary amine | None | Tertiary amine | Tertiary amine | Tertiary amine | Tertiary amine | Tertiary amine | Tertiary amine | Tertiary amine | None | None | None | Tertiary amine | Tertiary amine |
| Cell proliferation (change in cell count: day 6/day 1) | 2 | 5 | 5 | 5 | 4 | 2 | 2 | 4 | 4 | 2 | 2 | 1 | 1 | 2 |
| Initial adhesion (adsorbed cell count on day 1) | 2 | 4 | 5 | 5 | 5 | 3 | 2 | 4 | 5 | 2 | 1 | 1 (standard) | 2 | 4 |
| Particle aggregation | 4 | 4 | 4 | 4 | 4 | 4 | 4 | 5 | 4 | 5 | 4 | 5 | 5 | 1 |

(continued)

| | Ex. 17 | Ex. 18 | Ex. 19 | Ex. 20 | Ex. 21 | Ex. 22 | Ex. 23 | Ex. 24 | Ex. 25 | Ex. 26 | Ex. 27 | Comp. Ex. 1 | Comp. Ex. 2 | Comp. Ex. 3 |
|---|---|---|---|---|---|---|---|---|---|---|---|---|---|---|
| Cell adhesion inside carrier | 3 | 4 | 5 | 5 | 5 | 4 | 3 | 5 | 5 | 4 | 2 | 2 | 2 | 1 |
| Overall score | 11 | 17 | 19 | 19 | 18 | 13 | 11 | 18 | 18 | 13 | 9 | 9 | 10 | 8 |

INDUSTRIAL APPLICABILITY

The porous cellulose particles of the invention, when used as a culture carrier (culturing microcarrier), allow cells to be efficiently adsorbed and infiltrate during the initial stage of culturing when substrate adhesion between the cells is still undeveloped, while also reducing shedding of cells from the particles by the effects of shear stress even after high density culturing, and allowing the cells to be highly efficiently grown.

**Claims**

1.  Porous cellulose particles composed of cellulose,

    having collagen on the porous cellulose particle surfaces,
    wherein the fixed amount of the collagen in the dry state is 10 $\mu$g/g to 25 mg/g (0.001 to 2.5 wt%) per 1 g of porous cellulose particles.

2.  The porous cellulose particles according to claim 1, wherein the porous cellulose particles have numerous voids, with a mean opening size of 5 $\mu$m to 100 $\mu$m.

3.  The porous cellulose particles according to claim 1 or 2, wherein the porous cellulose particles form a continuous pore structure with adjacent voids mutually communicating by openings in the membranes dividing them.

4.  The porous cellulose particles according to claim 1 or 2, wherein the mean particle size of the porous cellulose particles is 100 $\mu$m to 1000 $\mu$m.

5.  The porous cellulose particles according to claim 1 or 2, wherein the sphericity of the porous cellulose particles is 0.7 or higher.

6.  The porous cellulose particles according to claim 1 or 2, wherein the specific surface area of the porous cellulose particles during drying is 0.3 m$^2$/g to 4.4 m$^2$/g.

7.  The porous cellulose particles according to claim 1 or 2, wherein the fixed amount of the collagen in the dry state is 1 mg/g to 10 mg/g (0.1 wt% to 1.0 wt%) per 1 g of porous cellulose particles.

8.  The porous cellulose particles according to claim 1 or 2, wherein the cellulose surfaces are modified with a cationic substituent.

9.  The porous cellulose particles according to claim 8, wherein the cationic substituent is at least one selected from the group consisting of primary amino, secondary amino and tertiary amino groups.

10. The porous cellulose particles according to claim 1 or 2, wherein the charge capacity of the porous cellulose particles is 0 mmol/g to 5.0 mmol/g.

11. The porous cellulose particles according to claim 1 or 2, wherein the collagen is fiberized collagen.

12. The porous cellulose particles according to claim 1 or 2, wherein the collagen is crosslinked collagen.

13. The porous cellulose particles according to claim 1 or 2, wherein the cellulose composing the porous cellulose particles is copper-ammonia regenerated cellulose.

14. The method for producing porous cellulose particles according to claim 1 or 2, the method comprising the following steps:

    a step of impregnating the porous cellulose particles with a collagen solution;
    a step of neutralizing the collagen solution to pH 6 to 8;
    a dewatering step in which the collagen solution is removed; and
    a drying step in which the porous cellulose particles are dried.

# EP 4 722 274 A1

## INTERNATIONAL SEARCH REPORT

International application No.

**PCT/JP2024/019915**

### A. CLASSIFICATION OF SUBJECT MATTER

*C08J 3/12*(2006.01)i; *C08B 11/145*(2006.01)i; *C08J 9/42*(2006.01)i; *C12M 1/00*(2006.01)i
FI:   C08J3/12 Z CEP; C08J9/42; C08B11/145; C12M1/00 C

According to International Patent Classification (IPC) or to both national classification and IPC

### B. FIELDS SEARCHED

Minimum documentation searched (classification system followed by classification symbols)

C08J3/12; C08B11/145; C08J9/42; C12M1/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2022-63983 A (ASAHI KASEI KABUSHIKI KAISHA) 25 April 2022 (2022-04-25) claims, paragraphs [0012]-[0022], examples | 1-13 |
| A | | 14 |
| Y | JP 2006-511219 A (FUJI PHOTO FILM B.V.) 06 April 2006 (2006-04-06) claims, paragraphs [0007]-[0008], [0027]-[0028], examples | 1-13 |
| A | | 14 |
| Y | JP 8-199481 A (MITSUBISHI RAYON CO.) 06 August 1996 (1996-08-06) claims, paragraph [0006], examples | 1-13 |
| A | | 14 |
| Y | JP 61-282307 A (KANEBO LTD.) 12 December 1986 (1986-12-12) claims, page 2, lower left column, lines 5-12, examples | 1-13 |
| A | | 14 |

☑ Further documents are listed in the continuation of Box C.     ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **08 August 2024** | **20 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

| International application No. |
| --- |
| **PCT/JP2024/019915** |

**C.      DOCUMENTS CONSIDERED TO BE RELEVANT**

| Category* | Citation of document, with indication, where appropriate, of the relevant passages | Relevant to claim No. |
| --- | --- | --- |
| A | WO 2021/250583 A2 (MESOBLAST INT SARL) 16 December 2021 (2021-12-16) entire text | 1-14 |
| A | JP 49-118998 A (HYOGO PREFECTURE) 13 November 1974 (1974-11-13) entire text | 1-14 |
| A | JP 58-58041 A (MITSUBISHI MINING & CEMENT CO., LTD.) 06 April 1983 (1983-04-06) entire text | 1-14 |

Form PCT/ISA/210 (second sheet) (July 2022)

**INTERNATIONAL SEARCH REPORT**
**Information on patent family members**

International application No.

**PCT/JP2024/019915**

| Patent document<br>cited in search report | | | Publication date<br>(day/month/year) | Patent family member(s) | Publication date<br>(day/month/year) |
|---|---|---|---|---|---|
| JP | 2022-63983 | A | 25 April 2022 | (Family: none) | |
| JP | 2006-511219 | A | 06 April 2006 | US 2007/0004034 A1<br>claims, paragraphs [0007]-<br>[0008], [0027]-[0028],<br>examples | |
| JP | 8-199481 | A | 06 August 1996 | (Family: none) | |
| JP | 61-282307 | A | 12 December 1986 | (Family: none) | |
| WO | 2021/250583 | A2 | 16 December 2021 | US 2023/0220349 A1<br>entire text | |
| JP | 49-118998 | A | 13 November 1974 | (Family: none) | |
| JP | 58-58041 | A | 06 April 1983 | (Family: none) | |

**EP 4 722 274 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2208331 A